# EUROPEAN PATENT APPLICATION

(11) **EP 3 903 831 A1**
(43) Date of publication of application: **03.11.2021**
(21) Application number: 19901524.9
(22) Date of filing: 08.05.2019
(51) Int. Cl.: A61K 49/10, A61B 5/055

(54) **MAGNETIC RESONANCE IMAGING DRUG CONTAINING A DEUTERATED NATURAL BRANCHED-CHAIN AMINO ACID, AND DIAGNOSTIC METHOD USING SAID DRUG**

(30) Priority: 29.12.2018 RU 2018147615
(71) Applicant: Solvex Limited Liability Company, Moscow 143026 (RU)
(72) Inventor: LESIV, Aleksei Valerevich, Moskovskaya obl., 141700 (RU); IVASHKIN, Pavel Evgenevich, Moscow, 125430 (RU); KOSENKOV, Aleksei Viktorovich, Moscow, 119602 (RU); POLSHAKOV, Vladimir Ivanovich, Moscow, 119333 (RU); KISELEVSKII, Mikhail Valentinovich, Moscow, 125319 (RU); KOROL', Boris Natanovich, Moskovskaya obl., 143085 (RU)
(74) Representative: Berggren Oy
(86) International application number: PCT/RU2019/000324
(87) International publication number: WO 2020/139126

(57) **Abstract**

The invention relates to the means for magnetic resonance imaging of oncological diseases and other diseases, accompanied by a locally altered level of absorption of nutrients by cells. The invention is the diagnostic drug containing at least one deuterated derivative of a natural branched-chain amino acid, as well as the diagnostic method based on the use of this diagnostic drug. The method of the invention includes performing magnetic resonance imaging and / or deuterium magnetic resonance spectroscopy after administration of the diagnostic drug in a time sufficient for the accumulation of the diagnostic drug in a target area of a subject's body. The proposed method allows to carry out a highly informative diagnostics of oncological diseases and other diseases, accompanied by a locally altered level of absorption of nutrients by cells.

## Description

### Technical field

The invention relates to medicine, namely to the means for magnetic resonance imaging.

### Prior art

Non-invasive diagnostics of diseases, including early diagnostics, is a key priority area in healthcare. One of the informative methods for the diagnostics of diseases is magnetic resonance imaging (MRI).

Most types of MRI used in clinical practice are based on recording the magnetic resonance signal of protons (¹H nuclei) contained in a human body water. ¹H MRI provides a high degree of anatomical detailing. At the same time, it is known from the clinical practice that MRI does not always provide unambiguous data about the nature of a disease. In particular, in oncology, a reliable distinction between malignant tumors and non-life-threatening benign formations, foci of inflammation, etc. remains challenging [see, for example: Baltzer, P.A.T. et al. Am. J. Roentenology, 2010, 194, 1658-1663; Shahid, H. et al. Appl. Radiol. 2016, 45, 7-13.]. In this regard, early diagnosis of oncological diseases is also difficult, since the risk of a false-positive result is high.

The main method to make ¹H MRI more informative is to use contrast agents that change the relaxation time of water protons in their environment [Topics in Current Chemistry, Contrast Agents I, Magnetic Resonance Imaging. Krause, W. (Ed.), Springer, 2002]. A wide range of contrast agents are known for being used in MRI diagnostics, including the commercially available Omniscan^{®}, Magnevist^{®}, ProHance^{®} and Clariscan^{®}, which are gadolinium complexes, as well as Feridex^{®} and Resovist^{®}, which are aqueous suspensions of stabilized magnetic nanoparticles. In addition to enhancing the contrast of images, these substances allow the assessment of perfusion.

An alternative to ¹H MRI with contrast agents is a registration of the signal from other nuclei, in particular, methods using isotopes ³¹P, ¹³C, ¹⁹F, ²³Na are at different stages of their clinical trials.

Deuterium (²H) is a natural, non-radioactive isotope of hydrogen, the content of which in biological objects is 0.0156% of the total amount of hydrogen.

Document US5042488 showed the possibility of registering the deuterium signal after injection of D₂O and 1- deuteroglucose *in vivo* (in rat's liver).

Document US20030211036 A1 proposed a method for measuring perfusion in selected tissue sections using isotopically labeled compounds (e.g. D₂O) by analogy with paramagnetic contrast agents.

Document US20100322865 A1 describes the use of metabolic water precursors to estimate metabolic rate. 1,2,3,4,5,6,6 -deuterated glucose is mentioned as an example of the metabolic precursor of HOD. Within the framework of the described invention, only NMR signals on metabolic water deuterium and aliphatic chain of fatty acids are recorded, and there are no NMR signals of deuterated glucose.

From the work [Washburn et al., Nucl. Med. 1978, 19, 77-83] it is known that racemic 1-¹⁴C-valine, as well as 1-¹¹C-valine accumulate mainly in the pancreas of animals. At the same time, no application of isotopically labeled valine derivatives for non-invasive diagnosis of diseases is known from the prior art.

¹⁴C-Leucine is widely used in biochemical scientific researches as an indicator of the protein synthesis rate (leucine is included in all proteins synthesized in a given tissue). This application typically requires invasive sampling of target tissues.

Valine, leucine and isoleucine belong to the group of branched-chain amino acids (BCAA). Members of this group have a certain biochemical similarity, since they can penetrate cell membranes through the same transporters, and the initial stages of their metabolism are catalyzed by the same enzymes (transaminase and decarboxylase of branched-chain amino acids).

Despite the widespread clinical application of ¹H MRI technology, there remains a need for developing new, more effective methods of MRI diagnostics.

### Disclosure of the invention

The goal of this invention is to develop a new effective diagnostic drug for diagnosing diseases by means of MRI and / or MR spectroscopy and a diagnostic method involving the use of the specified drug.

The technical result of this invention is to create a new and effective diagnostic drug that can be used for non-invasive diagnosis of diseases and pathological processes accompanied by a locally altered (increased or decreased) level of nutrients absorption by cells, in particular, oncological diseases, by means of magnetic resonance imaging and / or deuterium magnetic resonance spectroscopy.

This technical result is achieved through the development of the diagnostic drug that is compatible with the fundamental physical limitations of the nuclear magnetic resonance method (MRI or MR spectroscopy). It is known that the gyromagnetic ratio of the deuterium nucleus is 6.5 times less than that of protium. As a consequence, the detection sensitivity of the deuterium signal is approximately 0.01 (i.e. 1%) of the detection sensitivity of the protium signal [Biological Magnetic Resonance, Volume 11, In Vivo Spectroscopy. Berliner L.J., Reuben, J. (Eds.), Springer, 1992]. A weak signal can be recorded by averaging the signal of several identical scans. However, such averaging requires an increase in the scan time, and the signal-to-noise ratio increases non-linearly (proportionally,√*n* where n is the number of scans; for example, if the scan takes 10 minutes, and the signal-to-noise ratio should be increased by 10 times, an increase in the scan time will be required by 100 times, i.e. up to 16 hours). At that, the duration of scanning in living organisms is limited by both pharmacokinetics of the diagnostic drug and practical applicability in clinical practice (the examination should not take more than 1-2 hours) and the need for a patient to stay immobile during the entire scanning period. The intensity of the magnetic resonance signal also depends on the strength of the magnetic field. Since MR tomographs with a magnetic field strength of no more than 7T are currently approved for clinical application, only those diagnostic drugs that provide a sufficient signal intensity at 7T are practically applicable.

Thus, the deuterated compound in the diagnostic drug of the invention should have such a set of physicochemical and biological properties that ensures both the selective accumulation of deuterium and the maintenance of its concentration in the target tissues for a time sufficient for signal recording. The lower the concentration of the deuterated compound achieved in the tissue, the longer it is required to average the signal and, therefore, the longer the deuterated compound is to be in the tissues, maintaining a selective distribution. On the other hand, increasing the dose of the diagnostic drug is not a universal solution, since its excretion can be accelerated (in particular, by exceeding the reabsorption capacity of the kidneys), the risk of toxicity increases, the selectivity of accumulation in various tissues decreases, moreover the dose is limited by solubility.

For the most chemical compounds, including amino acids, there are no data on the maximum achievable non-toxic concentrations in tissues in case of pathology. As a consequence, the development of the diagnostic drug that meets the above criteria requires experimental evidence of its applicability in ²H MRI and / or *in vivo* MR spectroscopy.

The technical result of the present invention is also the development of a new effective and informative method for diagnosing diseases and pathological processes accompanied by a locally altered (increased or decreased) level of nutrients absorption by cells, in particular, oncological diseases, by means of magnetic resonance imaging and / or deuterium magnetic resonance spectroscopy including the introduction of the diagnostic drug of the invention, which is capable of accumulating in the target tissues and organs (in particular, in the tumor tissue) in a concentration sufficient to register an informative deuterium tomogram or ²H-NMR spectrum *in vivo.* Additional technical results in the implementation of the invention are the ability to obtain information about the level of perfusion at different points of the scanned region, information about the structure of a tumor, its boundaries, about the malignancy or benignity of the tumor. Another additional technical result is the possibility of assessing the local rate of metabolic processes in the scanned region, which, in turn, makes it possible to assess the level of a metabolic activity and / or cell proliferation, the rate of tumor growth and is an additional parameter that increases the reliability and validity of the diagnostics.

The method of the invention is also characterized by the fact that it is carried out without the harmful effects of ionizing radiation (typical, for example, for CT, PET, SPECT imaging), which in turn increases the safety of examinations, makes it possible to conduct more frequent repeated examinations, in particular, makes the method attractive for the pediatrics. The invention is aimed at obtaining a diagnostic information similar to the method of positron emission tomography or single-photon emission computed tomography (deviation of the level or rate of drug accumulation in pathological tissue from the norm or from the values achieved in the surrounding parts of the same tissue / the same organ), and yet eliminates the risks associated with ionizing radiation of radiopharmaceuticals. In addition, unlike radiopharmaceuticals for PET, the production of deuterated drugs of the invention is not limited to the synthesis and logistics of small batches of short lived isotopes.

Specified technical results are achieved through the development of the diagnostic drug comprising a deuterated derivative of a natural branched-chain amino acid and / or a pharmaceutically acceptable salt or mixture thereof for the diagnosis of diseases by magnetic resonance imaging and / or deuterium magnetic resonance spectroscopy.

Thus, the first aspect of the invention is the diagnostic drug comprising at least one compound selected from a deuterated derivative of a natural branched-chain amino acid and / or a pharmaceutically acceptable salt of a deuterated derivative of a natural branched-chain amino acid for the diagnosis of diseases or pathological processes by means of the magnetic resonance imaging and / or deuterium magnetic resonance spectroscopy.

In some embodiments of the invention, the diagnostic drug additionally comprises at least one pharmaceutically acceptable excipient. In some specific cases, the pharmaceutically acceptable excipient is a carrier, filler and / or diluent.

In some embodiments of the invention, a deuterated derivative of a natural branched-chain amino acid and / or a pharmaceutically acceptable salt thereof, along with deuterium atoms bonded to carbon atoms, contains deuterium atoms partially or completely replacing mobile hydrogen atoms bonded to oxygen and / or nitrogen atoms.

In some embodiments of the invention, the diagnostic drug comprises
- a deuterated derivative of a natural branched-chain amino acid containing deuterium atoms bonded to carbon atoms in more than one position, or a pharmaceutically acceptable salt thereof; or
- a mixture of deuterated derivatives of natural branched-chain amino acids and / or their pharmaceutically acceptable salts, with deuterated derivatives containing deuterium atoms in structurally nonequivalent positions.

In some embodiments of the invention, a deuterated natural branched-chain amino acid derivative is a deuterated valine or deuterated leucine or deuterated isoleucine.

In some specific embodiments of the invention, the deuterated valine is valine-4,4,4-d₃, valine-4,4,4,4',4',4'-d₆, valine-3,4,4,4,4',4',4'-d₇, valine-2,4,4,4,4',4',4'-d₇, valine-2,3,4,4,4,4',4',4'-d₈.

In some specific embodiments, the deuterated leucine is leucine-5,5,5-d₃, leucine-5,5,5,5',5',5'-d₆, leucine-4,5,5,5,5',5',5'-d₇, leucine-3,3,5,5,5,5',5',5'-d₈, leucine-3,3,4,5,5,5,5 ',5',5'-d₉, leucine-2,5,5,5,5',5',5'-d₇, leucine-2,3,3,5,5,5,5', 5', 5'-d₉, leucine-2,4,5,5,5,5',5', 5'-d₈, leucine-2,3,3,4,5,5,5,5',5',5'-d₁₀.

In some specific embodiments of the invention, the deuterated isoleucine is 2-amino-3-(CD₃) pentanoic acid, 2-amino-3-(CD₃) pentanoic-5,5,5-d3 acid, 2-amino-3-(CD₃) pentanoic-4,4,5,5,5-d5 acid, 2-amino-3-(CD₃) pentanoic-2,5,5,5-d4 acid, 2-amino-3-(CD₃) pentanoic-2,3,5,5,5-d₅ acid, 2-amino-3-(CD₃) pentanoic-3,4,4,5,5,5-d₆ acid, 2-amino-3-(CD₃) pentanoic-2,3,4,4,5,5,5-d₇ acid, 2-amino-₃-methylpentanoic-5,5,5-d₃ acid.

In some embodiments of the invention, the deuterated derivative is wholly or predominantly represented by one enantiomer. In some specific embodiments of the invention, the deuterated derivative is wholly or predominantly represented by the enantiomer with 2S configuration.

In some embodiments of the invention, the diagnostic drug comprises a mixture of at least two different compounds selected from a deuterated natural branched-chain amino acid derivative and / or a pharmaceutically acceptable salt of a deuterated natural branched-chain amino acid derivative. In some specific embodiments of the invention, the diagnostic drug comprises a mixture of at least two compounds selected from valine-4,4,4,4 ', 4', 4'-d₆, valine-2,4,4,4,4',4',4'-d₇, leucine-5,5,5,5',5',5'-d₆, leucine-3,3-d₂.

In specific embodiments of the invention, the diagnostic drug may contain an inhibitor of branched-chain amino acid metabolism, in particular, an inhibitor of branched-chain amino acid transaminase. For instance, selected from gabapentin (A. Goldlust et al. Epilepsy Res. 1995, 22(1), 1-11), benzimidazoles (H. Deng et al. ACS Med. Chem. Lett. 2016, 7(4), 379-384), but not limited to them.

Another aspect of the invention is a method for diagnosing a disease or pathological process in a subject, comprising the following steps:
- administering a diagnostic drug of the invention to a subject;
- conducting magnetic resonance imaging and / or deuterium magnetic resonance spectroscopy after the administration of the diagnostic drug in a time sufficient for its accumulation in a target tissue, to obtain, respectively, a tomogram (deuterium tomogram) and / or NMR spectrum (spectra);
- diagnosing the presence or absence of a disease based on the observed deuterium nuclei signal intensity, reflecting the level of accumulation of the diagnostic drug.

In preferred embodiments of the invention, the diagnosed disease or pathological process is accompanied by a locally altered (increased or decreased) level of nutrients absorption by cells.

In some embodiments of the invention, the pathological process is an inflammatory process, an infectious process, a process accompanied by active regeneration, a disease associated with ischemia of organs and tissues, graft rejection reaction, an autoimmune disease. In some other embodiments of the invention, the disease is an oncology disease. In some specific embodiments of the invention, an oncology disease is a solid tumor or tumor metastases, including lymph node metastases.

In some embodiments of the invention, the presence or absence of a disease is diagnosed by comparing the signal intensity of deuterium nuclei in a subject undergoing an examination with the typical signal intensity observed in healthy subjects in a target tissue or organ. In some other embodiments of the invention, the presence or absence of a disease is diagnosed based on a comparison of the signal intensity of deuterium nuclei in regions corresponding to normal and abnormal tissue according to additional medical examination results. In some embodiments of the invention, the presence or absence of a disease is diagnosed based on a combination of the above comparisons.

In some embodiments of the invention, at least one additional medical examination is carried out, selected from magnetic resonance imaging on nuclei other than deuterium nuclei, ultrasound examination, computed tomography, radiography, palpation, biopsy, analysis of biological fluids for tumor markers, radionuclide diagnostics and / or visual observation. In some embodiments of the invention, additional medical examination is carried out before diagnosing a disease or pathological process using magnetic resonance imaging and / or deuterium magnetic resonance spectroscopy, as described above. In some other specific embodiments of the invention, additional medical examination is carried out after diagnostics of a disease or pathological process by magnetic resonance imaging and / or deuterium magnetic resonance spectroscopy, as described above.

In some specific embodiments of the invention, the presence or absence of a disease or pathological process is diagnosed based on a comparison of a deuterium tomogram of a subject undergoing an examination with an image obtained as a result of magnetic resonance imaging of a subject on the protium nuclei.

In some embodiments of the invention, a spatial distribution of a region with increased accumulation of the diagnostic drug on a deuterium tomogram, is used to make a conclusion about the spatial structure of a tumor.

In specific embodiments of the invention, the deuterium signal intensity on a deuterium tomogram and / or the NMR spectrum (spectra) in the area of increased accumulation of the diagnostic drug is used to make a conclusion about the malignancy or benignity of a tumor.

In some specific embodiments of the invention, the rate of change in the intensity of the deuterium signal on a deuterium tomogram and / or the NMR spectrum (spectra) after its administration is used to make a conclusion about the level of perfusion at different points of the scanned region.

Specific embodiments of the method for diagnosing a disease or pathological process in a subject related to the invention also include all embodiments of the invention in regard to the diagnostic drug described above.

In some embodiments of the method for diagnosing a disease or pathological process in a subject, the diagnostic drug to be administered comprises
- a deuterated derivative of a natural branched-chain amino acid containing deuterium atoms bonded to carbon atoms in more than one position, or a pharmaceutically acceptable salt thereof; or
- a mixture of deuterated derivatives of natural branched-chain amino acids and / or their pharmaceutically acceptable salts, in which deuterated derivatives are present containing deuterium atoms in structurally nonequivalent positions.
at the same time, based on a comparison of the signal intensity of deuterium nuclei in structurally nonequivalent positions of the deuterated derivative (s) in a target area, the local metabolic rate of natural branched-side amino acids is assessed, which allows more accurate diagnostics, in particular, to assess the growth rate or malignancy of tumors.

In some specific cases of the above-described embodiments of the invention, the deuterated derivative of a natural branched-chain amino acid is
- a mixture of various deuterated valine derivatives; or
- a mixture of various deuterated leucine derivatives; or
- a mixture of various deuterated isoleucine derivatives; or
- a mixture of various deuterated derivatives of valine, leucine and / or isoleucine.

In some embodiments of the invention, the diagnostic drug is administered to a subject per os. In some other embodiments of the invention, the diagnostic drug is administered to a subject parenterally.

### Brief description of the drawings

**Figure 1****.** a) deuterium tomogram (on the left) and ²H spectrum (on the right) of the sample with valine-4,4,4,4 ", 4', 4'-d₆; b) deuterium tomogram (on the left) and ²H spectrum (on the right) of the sample with leucine-5,5,5,5 ', 5', 5'-d₆.
**Figure 2****.** Tomograms of the mouse with 4T1 breast carcinoma after injection of 20 mg valine-4,4,4,4 ', 4', 4'-d₆: a) ²H tomograms obtained at different points in time after injection (surface coil is indicated by a dotted line); b) ²H tomogram (on the left), overlay of ²H and ¹H tomograms (in the center), ¹H tomogram (on the right).
**Figure 3****.** Tomograms of the mouse with 4T1 breast carcinoma 40 minutes after injection of 25 mg of leucine-5,5,5,5 ', 5', 5'-d₆.
**Figure 4****.** Tomograms of the mouse with 4T1 breast carcinoma after administration of: a) 30 mg of L-alanine-3,3,3-d₃; b) 10 mg of L-phenylalanine ― β,β,2,3,4,5,6-d₇.

### Definitions and terms

For a better understanding of the present invention, below are some of the terms used in the present description of the invention.

For the purposes of description of this invention, the terms **"includes"** and **"including"** are interpreted to mean "includes, among other things". These terms are not intended to be construed as "consists only of".

The term **"subject"** encompasses all mammalian species, preferably humans.

The term **"deuterated derivative"** in this document means a compound containing deuterium bound to carbon in an amount exceeding its natural content in at least one position. In specific embodiments of the invention, the deuterium content, at least in one position, exceeds 10%, in other specific embodiments - 90%. "A mixture of at least two different deuterated derivatives" means a mixture of compounds containing deuterium in different positions, or containing different amounts of deuterium in the same position. The symbol "d" ("D") in this document denotes a hydrogen atom represented by the isotope ²H in a proportion exceeding its natural content.

Deuterated branched-chain amino acids derivatives include valine-4,4,4-D₃, valine-4,4,4,4 ', 4', 4'-D₆, valine-3,4,4,4,4',4',4'-D₇, valine-2,4,4,4,4',4',4'-D₇, valine-2,3,4,4,4,4', 4', 4'-D₈, leucine-5,5,5-D₃, leucine-5,5,5,5',5',5'-D₆, leucine-4,5,5,5,5',5',5'-D₇, leucine-3,3,5,5,5,5',5',5'-D₈, leucine-3,3,4,5,5,5,5',5',5'-D₉, leucine-2,5,5,5,5',5',5'-D₇, leucine-2,3,3,5,5,5,5',5',5'-D₉, leucine-2,4,5,5,5,5',5',5'-D₈, leucine-2,3,3,4,5,5,5,5',5', 5'-D₁₀, 2-amino-3- (CD₃) pentanoic acid, 2-amino-3- (CD₃) pentanoic-5,5,5-d₃ acid, 2-amino-3- (CD₃) pentanoic-4,4,5,5,5-d₅ acid, 2-amino-3- (CD₃) pentanoic-2,5,5,5-d₄ acid, 2-amino-3- (CD₃) pentanoic-2, 3,5,5,5-ds acid, 2-amino-3- (CD₃) pentanoic-3,4,4,5,5,5-d₆ acid, 2-amino-3- (CD₃) pentanoic-2, 3,4,4,5,5,5-d₇ acid, 2-amino-3-methylpentanoic-5,5,5-d₃ acid, but not limited to them.

The term **"voxel"** in this document refers to the minimum volume element of a scan area, which corresponds to a specific value of deuterium signal intensity or a specific local spectrum.

**"Structurally nonequivalent"** is defined herein as any two positions in the structure of a natural branched-chain amino acid, denoted by different symbols in the following figure.

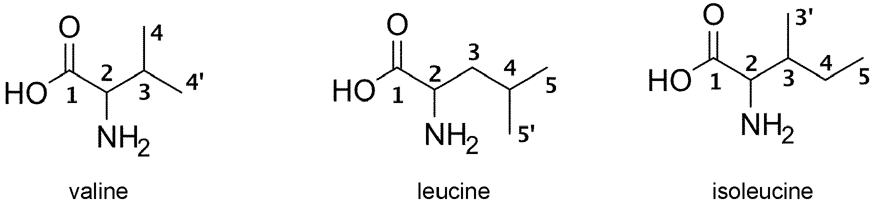

Also, any positions in the structure of one branched-chain amino acid with respect to any positions in the structure of another branched-chain amino acid (in particular, any positions in the structure of valine with respect to any positions in the structure of leucine) are considered structurally nonequivalent. For example, structurally nonequivalent are: a) positions 4 and 4 'in L-valine-4,4,4,4',4',4'-d₆; b) position 4 in L-valine-4,4,4,4',4',4'-d₆ and position 3 in L-valine-3,4,4,4,4',4',4'- d₇; c) position 4 in L-valine-4,4,4,4',4',4'-d₆ and position 4 in leucine-4,5,5,5,5',5', 5'-d₇.

As used herein, the term **"pharmaceutically acceptable salt"** refers to those salts that are suitable for use in contact with human and animal tissues without undue toxicity, irritation, allergic reaction, etc., and meet a reasonable risk-benefit ratio. Pharmaceutically acceptable salts of amines, carboxylic acids, phosphonates, and other types of compounds are well known in the medicine. Salts can be prepared *in situ* during the isolation or purification of the compounds of the invention, and can also be prepared separately by reacting of a free acid or free base of the compound of the invention with a suitable base or acid, respectively. An example of pharmaceutically acceptable, non-toxic acid salts are salts of the amino group formed with inorganic acids such as hydrochloric, phosphoric, or organic acids such as acetic, oxalic, maleic, fumaric, tartaric, succinic, ascorbic, citric, butyric, lactic, gluconic acids , or obtained by other methods used in this field, for example, using ion exchange. Typical salts of alkali and alkaline earth metals contain sodium, potassium, calcium, magnesium and others. In addition, pharmaceutically acceptable salts may contain, if required, non-toxic ammonium, quaternary ammonium and amine cations obtained using counterions such as halides, hydroxides, carboxylates, sulfates, phosphates, and others.

The diagnostic drug of the invention may include one or more of any pharmaceutically acceptable excipients suitable for a particular dosage form, in particular, any carriers, diluents and / or fillers, such that can be administered to a patient's body together with a compound that constitutes the essence of this invention, and which do not destroy this compound, and are non-toxic when administered. Non-limiting specific examples of pharmaceutically acceptable excipients include sodium chloride, glucose, sweeteners, food flavors, colors, and others.

### Detailed disclosure of the invention

The successful diagnostics of a disease using ²H MRI or MR spectroscopy is based on the ability of a particular deuterated compound to accumulate selectively in various tissues and, at the same time, to create a signal with an intensity sufficient from the point of view of the fundamental physical limitations of nuclear magnetic resonance methods (gyromagnetic ratio, relaxation parameters of deuterium nuclei). In this case, the intensity of the deuterium signal:
a) is proportional to the concentration of deuterium achieved in a tissue (depends on the dose, the kinetics of membrane transport, the concentrating ability of the tissue),
b) is proportional to the square root of the scan time (the time is limited by the rate of elimination and / or metabolism of the deuterated compound, as well as the need for a patient to stay immobile during the entire scanning procedure),
c) depends on the relaxation time T₁ (determines the maximum averaging rate and, consequently, the intensity of the total signal received per unit of time; for different compounds it differs by several times: see. Biological Magnetic Resonance, Volume 11, In Vivo Spectroscopy. Berliner L.J., Reuben, J. (Eds.), Springer, 1992).

Each chemical compound has unique pharmacokinetic parameters (in particular, the concentration of the compound and the rate of its change in the blood, various organs and tissues). In this case, the pharmacokinetics in a non-obvious way depends on a dose used (in particular, an excess of the renal reabsorption capacity for a particular compound can lead to its accelerated excretion). At that, the dose is limited by the toxicity and solubility of this compound. Experiments carried out by the authors, as well as references to the prior art, show that freely diffusing deuterated compounds such as D₂O do not show selective accumulation in various organs and tissues of a subject.

The authors have found that the deuterated natural branched-chain amino acid derivatives of the invention:
- are able to selectively accumulate in animal tissues at a concentration sufficient for visualization of various organs and tissues *in vivo,* including cancerous tumors, by ²H MRI or MR spectroscopy;
- possess pharmacokinetic properties that allow the use of non-toxic doses, while successfully registering ²H MRI and / or ²H NMR spectra;
- at concentrations sufficient for successful registration of ²H MRI and / or ²H NMR, they are characterized by rather slow excretion and metabolism, consistent with the time constraints of deuterium tomography and MR spectroscopy methods.

In turn, this allows for effective diagnosis of diseases and pathological processes accompanied by a locally altered (increased or decreased) level of nutrients absorption by cells, including determining the presence and localization of an oncological disease, by means of deuterium magnetic resonance imaging. At the same time, our experiments have demonstrated that other amino acids, in particular, glycine-d₂, L-alanine-3,3,3-d₃ and L-phenylalanine-β,β,2,3,4,5,6-d₇ do not have suitable physical-chemical and biological properties and, therefore, cannot be used for the diagnosis of diseases by ²H MRI or MR spectroscopy.

Isotopically labeled derivatives of valine and leucine (¹⁴C, ¹³C, ³H) were previously used to determine the rate of protein synthesis by analyzing the protein fraction isolated from a homogenate of various biological tissues (see: Attaix et al., Biochim. Biophys. Acta 1986, 882, 389-397; Goto et al., Chem. Soc. Bull, 1977, 25(7), 1574-1581). However, these studies lack data to suggest the possibility of achieving a concentration of valine or leucine that is not bound in proteins, as well as its low molecular weight metabolites in tissues in pathologies that are compatible with ²H MRI or NMR recording *in vivo.* From data of Goto et al. it also follows that the most part of ¹⁴C-valine accumulated in the liver and pancreas is included in the protein composition within 10 minutes after administration.

From the work [Washburn et al., Nucl. Med. 1978, 19, 77-83] it is known that at doses up to 5 mg / kg racemic 1-¹⁴C-labeled valine accumulates in the pancreas of animals. In this case, the absolute concentrations of ¹⁴C isotope achieved in different tissues can be calculated based on the presented data only for a dose of 0.021 mg / kg, which is approximately 500 times less than the dose required for the implementation of ²H MRI according to data received by us. At the same time, there are no literature data on the maximum achievable concentration of valine in animal tissues at doses of about 0.05-1 g / kg. As it can be seen from our data (Table 1), the distribution of deuterium in the methyl groups of valine-4,4,4,4',4',4'-D₆ in the organs of an animal differs from the distribution of the radioactive label observed for 1-¹⁴C- valine. The observed difference in distribution is due to two factors:
1) The dose of valine-4,4,4,4',4',4'-D₆ of the invention is more than 100 times higher than the maximum studied dose of 1-¹⁴C-valine. The efficiency of accumulation in a target tissue depends on the ratio of the intracellular and extracellular concentration of amino acids in animal or human cells. As is known from the prior art, this ratio depends on the extracellular concentration. Thus, in case of 2-aminoisobutyric acid and mouse thymus cells, this ratio falls from 100: 1 to 1: 1 with an increase in extracellular concentration from 10-⁶ to> 10-² mmol / L [Helmreich, E., Kipnis, DM 1962, 237, 8, 2582-2589]. Thus, as follows from the prior art, one would expect a sharp decrease in the selectivity of accumulation and leveling of contrast in various tissues with an increase in the dose by two orders of magnitude (i.e., when passing from 1-¹⁴C-valine to valine-d₆). In addition, the administration of high doses of amino acids (e.g., deuterated valine of the invention) *in vivo* leads to a disproportionate increase in the concentration of valine in various organs and tissues (Table 1).
2) The signal observed as a result of radioactive decay of 1-¹⁴C-valine is a superposition of signals from free valine, valine included in proteins, and other valine metabolites containing ¹⁴C isotope (for example, 1-¹⁴C-ketoisovaleric acid). The same work informs that ¹⁴C isotopic label is rapidly split off in the form of [¹⁴C] -CO₂ (elimination in the form of CO₂ reaches 28% in 60 minutes). On the other hand, the observed signal of deuterium methyl groups in the diagnostic drug of the invention includes the signal of isobutanoic acid (a decarboxylation product invisible in the case of 1-¹⁴C-valine) and at the same time does not contain the contribution of proteins (the relaxation parameters of proteins are not compatible with imaging in ²H MRI). Thus, the diagnostic drug of the invention and 1-¹⁴C-valine provide information on the distribution of various sets of compounds in the body.

Since valine-d₆ residues in proteins do not contribute to the ²H signal during the implementation of the method of the invention, the diagnostic drug based on valine-d₆ allows selective visualization of free valine and its low-molecular-weight metabolites without admixture of protein signal.

Radioactively labeled leucine derivatives have previously been used to assess the rate of protein synthesis (e.g.1-¹¹C-leucine: P.J. Hellyer et al. Neurolmage, 2017, 155, 209-216). As in the case of valine, the relaxation time of leucine in MRI change dramatically when included in the composition of proteins during synthesis, which makes it possible to selectively track the signal of free leucine and its low molecular weight metabolites (impossible in the case of methods based on a radioactive label).

A distinctive feature of deuterated derivatives of natural branched-chain amino acid is the ability to observe several deuterium signals at once in MRI or MR spectroscopy using a derivative or a mixture of derivatives containing deuterium atoms in several structurally nonequivalent positions at once, for example, valine-2,4,4,4,4 ', 4', 4'-d₇. It should be noted that in the process of stage-by-stage metabolism of branched-chain amino acids, structurally nonequivalent positions sequentially change the chemical shift of the corresponding deuterium atoms, and also selectively acquire the ability to exchange deuterium for water protium. As distinct from the methods based on radioactive isotopes, this allows, in addition to the level of accumulation of the deuterated derivative, to obtain information on the local metabolism of branched-chain amino acids based on the difference in the intensity of the drug deuterium signals. The intensity ratio of NMR signals of deuterium atoms in the α-position and in different positions of the branched-chain amino acid(s) in this case reflects the differences of the voxels of the scanning region under study in the rate of transamination (shown in Table 1) and the subsequent catabolism of branched-chain amino acid(s). Catabolism of branched-chain amino acids goes stage-by-stage: first, the α-position is oxidized (in this case, the deuterium atom in the α position is lost in the deuterated derivative), then the corresponding keto acid is decarboxylated, after which the process of stepwise oxidation of the fragment corresponding to positions 3-4 of valine and 3- 5 of leucine or isoleucine (in this case, deuterium atoms are lost in the corresponding positions in the deuterated derivative). Branched-chain amino acids are known to be used by cells for protein synthesis and are also an important source of energy. Thus, an increased local rate of branched-chain amino acid metabolism may indicate a locally increased proliferation rate, in particular, the growth or malignancy of a tumor or its certain parts. The use of such additional diagnostic information on the metabolism of branched-chain amino acids makes the diagnostic method of the invention more reliable. The intensity ratio of the NMR signals of deuterium atoms at different positions of deuterated derivatives of branched-chain amino acids can be measured both once per scan and several times.

The weight ratio of two different deuterated branched-chain amino acid derivatives in a single diagnostic product can range from 1: 1 to 1:10, but are not limited to them.

It is known from the prior art that different amino acids have different kinetics of accumulation in animal and human cells, and also differ dozens of times in the concentration gradient achieved in the equilibrium state [Johnstone, R.M., Scholefield, P.G., Adv. Cancer Res. 1965, 9, 143-226]. The behavior of amino acids *in vivo* is further complicated by homeostasis and directed transport at the level of the whole body. In particular, it is known that excess alanine released by the muscles is actively absorbed by the liver; at a physiological concentration, glutamine is actively absorbed by several types of cancerous tumors. Since deuterium tomography or spectroscopy involves the administration of doses several times higher than physiological, and different organs and tissues are characterized by different accumulation kinetics and capacities for various amino acids, it is not possible to predict the presence of selective accumulation of a specific amino acid without a direct experiment.

An important property of the deuterated component (branched-chain amino acid derivative) of the diagnostic drug of the invention is sufficient resistance to metabolic exchange of deuterium for protium *in vivo.* This exchange lowers the concentration of the deuterium label, while simultaneously increasing the background signal of heavy water (DOH), which is evenly distributed throughout the body due to rapid diffusion. This process leads to a decrease in the contrast of the image, and also prevents quantitive evaluation of deuterated component concentration by comparison with the intensity of natural DOH signal. Our studies have shown that some deuterated natural amino acids, in particular glycine-2,2-d₂ and L-alanine-3,3,3-d₃, cannot be used to obtain diagnostically significant images related to the invention, since they lose deuterium *in vivo* very quickly.

Due to low deuterium content in the body (0.015% hydrogen atoms), the background signals in ²H MRI are several orders of magnitude lower than in ¹H MRI. Thus, even at a low concentration of the diagnostic drug, its signal is not superimposed on the signals of natural background components. The development of similar methods using non-deuterated diagnostic drugs based on ¹H MRI is difficult due to the existence of a large number of background signals of natural low-molecular-weight compounds with an intensity comparable to the maximum achievable signal intensity of a non-deuterated diagnostic drug.

The effectiveness of the diagnostic drug is also determined by the sufficient number of deuterium atoms in the structure of the deuterated derivative. Thus, the diagnostic drug comprising a deuterated derivative of a natural branched-chain amino acid containing one or more CD₃ groups is a preferred embodiment of the present invention. The presence of such groups allows diagnostics using lower doses of the diagnostic drug, which leads to minimization of side effects.

The method of the invention makes it possible to diagnose, in particular, the presence or absence of an oncological disease, accompanied by the formation of solid tumors (both primary and metastatic) and / or metastases in the lymph nodes. Oncological diseases that can be diagnosed with deuterated diagnostic drugs include: breast cancer, lung cancer, prostate cancer, melanoma, brain cancer (including metastases from other tumors), kidney cancer, colon cancer, pancreatic cancer, ovarian cancer, uterine cancer, non-Hodgkin's lymphoma, liver cancer, sarcoma, but not limited to them. In addition to oncological diseases, the method of the invention can be used in the diagnosis of other diseases characterized by high metabolic activity or cell proliferation: for example, in case of transplanted organs and cells rejection, in autoimmune, inflammatory or infectious diseases, in liver damage accompanied by active regeneration. It is also possible to diagnose diseases that develop as a result of blood supply disturbance (ischemia) of various organs, for example, heart, brain, kidneys. Violation of the blood supply leads to a decrease in the rate of absorption and the achieved level of accumulation in these organs of amino acids, including branched-chain amino acids, as observed by ²H MRI or MR spectroscopy.

The method of the invention is based on the use of the deuterated diagnostic drug and registration of tomograms and / or NMR spectra at the deuterium frequency. The authors are not aware of the use of deuterated natural branched-chain amino acids for MRI diagnostics of diseases.

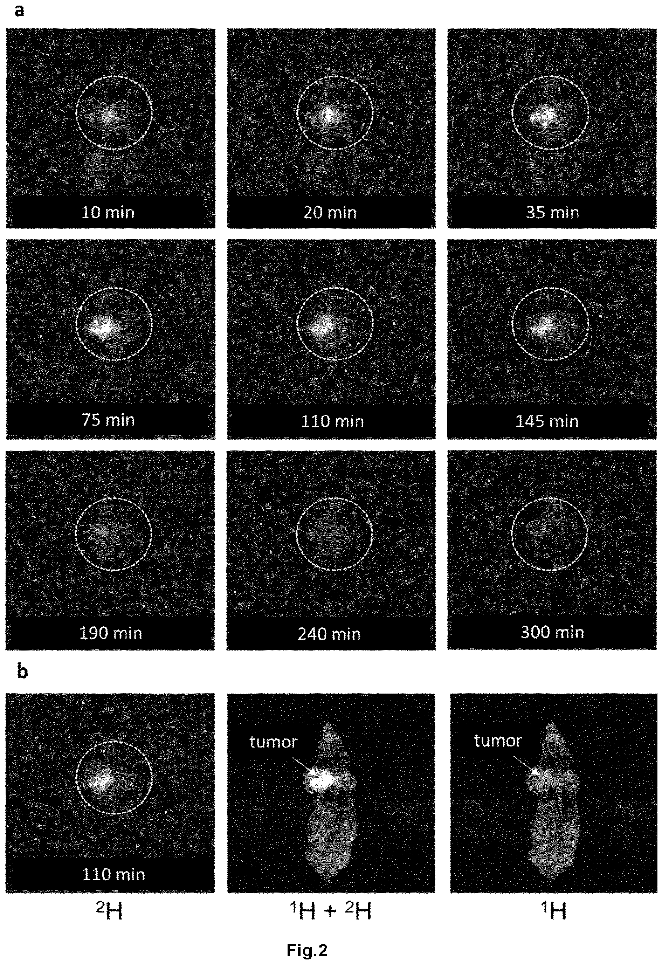

It is known that ¹H MRI by itself in many cases has insufficient diagnostic accuracy. The same applies to the MRI methods based on measurement of perfusion parameters (e.g., dynamic contrast-enhanced MRI). In contrast to perfusion methods, the diagnostic method of the invention provides data on the rate of membrane transport and the level of accumulation of branched-chain amino acids in cells, typical for PET or SPECT, and not available in traditional methods of implementing ¹H MRI. Thus, the method of the invention provides more accurate diagnostic information. In particular, in case of an oncological disease, the method of the invention makes it possible to assess the metabolic activity of a a target tissue, and, as a consequence, to conclude that a tumor is malignant or benign, and to assess its aggressivity. The signal of the diagnostic drug of the invention can be observed for at least 3 hours (see figure 2). The rate of change in the signal intensity in a cancer tumor and various internal organs (liver, spleen, pancreas, kidneys) during repeated scanning for up to 3 hours reflects the level of perfusion and metabolic activity of these tissues and organs, which can be used to make a more accurate diagnosis based on deuterium tomography and / or MR spectroscopy.

In specific cases of the embodiment, the spatial distribution of the deuterium signal of the diagnostic drug is used to make a conclusion about the spatial structure of a tumor.

In other specific embodiments of the invention, the intensity of the deuterium signal in a region with an increased content of the diagnostic drug is used to make a conclusion about the degree of malignancy / aggressivity of a tumor. Malignant tumors are characterized by a more active metabolism and increased activity of membrane transport. Thus, in tumors with a higher grade of malignancy, the signal intensity of deuterium will be higher.

In other specific embodiments of the invention, the perfusion in a target area is assessed by the rate of change in the deuterium signal intensity of the diagnostic drug. In regions with a high degree of perfusion, the rate of change in the deuterium signal with time (from the start of accumulation in a tumor tissue to complete elimination) is higher.

The diagnostic drug of the invention is administered in an amount effective for the diagnosis. An effective amount in this case means that amount of a compound (a deuterated derivative of a natural branched-chain amino acid and / or a pharmaceutically acceptable salt thereof) administered or delivered to a patient when the desired effect is most likely to occur - the possibility of carrying out the diagnostic method of the invention by magnetic resonance imaging and / or deuterium magnetic resonance spectroscopy. Due to the fundamental limitations of the deuterium MRI method, the amount of deuterated branched-chain amino acid derivative and / or its pharmaceutically acceptable salt cannot be ultra-low and is used in doses exceeding 10 mg / kg, for example 0.1-1.5 g / kg. In particular, when calculating the dose of the drugs for mammals of different species, it is usually not weight that is used, but the surface area of a body, which is nonlinearly dependent on weight. The exact amount required may vary from subject to subject, depending on a mammal species, age, body weight and general condition of a patient, the severity of a disease, and the method of the drug administration.

It is also known from the prior art that the half-life of the same compound can differ from one species to another (usually the half-life is longer in larger species), and therefore the optimal time between drug administration and scanning can differ significantly for different mammals. The optimal time between drug administration and scanning depends on the nature of a disease and the area of a subject's body being examined. The registration of the deuterium signal can be carried out, inter alia, until the end of the administration of the diagnostic drug and can be continued or repeated as long as the deuterium signal is present in the scanned region.

The diagnostic drug of the invention can be administered to a patient by any route of administration that is effective for the diagnosis, for example, it can be administered per os, parenterally, locally, etc.

In one of embodiments of the invention, the diagnostic process includes MRI and is carried out as follows:
a) In some embodiments of the invention, protium (¹H) MRI is carried out. Registration of ¹H MRI allows, firstly, to carry out anatomical assignment of the deuterium signal, and secondly, to identify regions with suspected pathology, in particular, a malignant neoplasm (in other embodiments, the region of ²H MRI can be determined in other ways, in particular, by means of ultrasound, computed tomography, radiography, palpation, biopsy, analysis of biological fluids for tumor markers, radionuclide diagnostics and / or visual observation);
b) the diagnostic drug is administered;
c) after a time, sufficient for the accumulation of the diagnostic drug in a target tissue of a subject, the tomogram is recorded at the frequency of the precession of the diagnostic drug deuterium nuclei;
d) the obtained deuterium tomograms are analyzed in order to find regions with abnormally high or low intensity and, therefore, corresponding to the selective accumulation of the diagnostic drug. In particular, it is possible to compare tomograms obtained on ¹H and ²H: if the abnormal regions on ¹H and ²H coincide, it can be said that there is a greater likelihood of the pathology.

In another embodiment of the invention, the diagnostic process includes conducting deuterium MR spectroscopy and is carried out as follows:
a) ¹H MRI is carried out, then the regions with suspected pathology, in particular, malignant neoplasm are identified (in other embodiments of the invention, the determination of the ²H MRI region can be carried out by other methods, in particular, by means of ultrasound examination, computed tomography, radiography, palpation, biopsy, analysis of biological fluids for tumor markers, radionuclide diagnostics and / or visual observation);
b) the diagnostic drug is administered;
c) after a time, sufficient for the accumulation of the diagnostic drug in a target tissue of a subject in voxels corresponding to a region with suspected pathology (for example, according to the results of ¹H MRI), the deuterium spectrum is recorded (in particular, using the methods of local spectroscopy); optionally, the spectrum is registered in adjacent voxels to compare the signal intensity;
d) the signal intensity in voxels corresponding to the area with suspected pathology is compared, in particular, with: (i) typical values for a given organ or tissue (which should be determined in advance in healthy subjects) and / or (ii) intensity in adjacent voxels corresponding to the same organ or tissue and free from abnormalities on ¹H MRI. An increased or decreased signal intensity suggests a selective accumulation of the diagnostic drug and, as a consequence, the presence of pathology, in particular, a malignant neoplasm.

The order of steps "a), b), c)" in both of the above embodiments of the invention can be changed to "b), a), c)" or "b), c), a)". It is also possible to register signals ¹H and ²H in parallel (i.e., simultaneous carrying out of stages "a)" and "c)").

In specific embodiments of the invention after identifying a region with suspected malignant formation, individual voxels are selected that lie both within and outside the suspicious region (in particular, a series of adjacent voxels lying on the same line crossing the border of a suspicious area can be selected). Registration of the integral signal of ²H or local ²H spectra in the selected voxels with subsequent comparison of their intensity makes it possible to quickly and with greater sensitivity to detect the regions where the diagnostic drug is accumulated.

MRI images and MR spectra can be obtained on any MRI scanner fitted with equipment for recording the deuterium signal.

A distinctive feature of deuterated derivatives of natural branched-chain amino acids is that these amino acids are natural nutrients and constituents of the human and animal body. This makes them safer when used as the diagnostic drug than unnatural amino acids. The examinations carried out by the authors of the invention indicate a good tolerance of the diagnostic drug by animals, the absence of visible side effects when used in the indicated doses on any MRI scanner fitted with equipment for registering a signal of deuterium. Branched-chain amino acids are known from the prior art to be safe when administered in high doses (LD₅₀> 5 g / kg). Considering the effective catabolism of deuterated branched-chain amino acids derivatives with the concomitant release of deuterium in the form of DOH (non-toxic in very high doses, up to the replacement of 10-30% of water in the body, and is also present in the body water at a concentration of about 1×10-² mol / L), we do not expect side effects associated with the introduction of multiple deuterium atoms.

In specific embodiments, the diagnostic drug may contain an inhibitor of branched-chain amino acid metabolism, in particular, an inhibitor of branched-chain amino acid transaminase. BCAT; an example of an inhibitor: Hu, L.Y. et al. Bioorg. Med. Chem. Lett. 2006, 16, 9, 2337-2340). Inhibition of the metabolism of branched-chain amino acids allows for a longer tomographic examination and / or lowering the dose of the diagnostic drug and / or to increase the sensitivity and, as a consequence, the reliability of the diagnostic method of the invention. The use of a selective inhibitor of individual transaminase isoforms of branched-chain amino acids as part of the diagnostic drug can be used to visualize the activity of individual isoforms in different parts of the scanned region.

The method of the invention is carried out without the harmful effects of ionizing radiation (typical, for example, for CT, PET, SPECT), which in turn increases the safety of examinations, makes it possible to conduct more frequent repeated studies, in particular, makes the method attractive for pediatrics.

The diagnostic method of the invention can be used, in particular, for the early diagnosis of malignant tumors of various localization, metastatic lesions, assessment of a tumor response to treatment and conclusions about the effectiveness of the therapy, to clarify the diagnosis based on the results of ¹H MRI and / or other diagnostic methods.

The method of the invention expands the existing possibilities of non-invasive diagnostics, and allows for effective diagnosis of oncological diseases.

Pharmaceutically acceptable salts of deuterated branched-chain amino acid derivatives have all the properties required to be used in the diagnostic drug of the invention.

### Implementation of the invention

The possibility of objective manifestation of the technical result when using the invention is confirmed by reliable data given in the examples containing experimental data obtained in the process of conducting research according to the methods applied in this field. The essence of the invention is illustrated by the figures.

It should be understood that these and all examples given in the application materials are not limiting and are given only to illustrate the present invention.

The examples given in this document serve to illustrate the principle of operation of the developed method and do not limit the range of doses used, as well as the range of time between the administration of the diagnostic drug and the registration of the deuterium signal, since, depending on the sensitivity and other parameters of the equipment used, a diagnosed disease and the nature of a subject (human or laboratory animal), the required doses and the time required for drug accumulation may differ. In particular, it is known from the prior art that the half-life of the same compound can vary between animal species, and that when changing from one animal to another or to humans, doses tend to scale in proportion to body surface area rather than a body weight. Registration of the deuterium signal can be made also before the end of the administration of the diagnostic drug. In addition, the above parameters for recording spectra and tomograms, including the signal accumulation time, are part of specific embodiments of the invention and may vary depending on the equipment used and specific diagnostic tasks.

### Synthesis of valine-4,4,4,4 ', 4', 4'-D₆

Valine-4,4,4,4 ', 4', 4'-D₆ was synthesized from the nickel (II) complex described in the literature (S) -BPB-Ni-Gly (Y. N. Belokon et al., Tetrahedron: Asymmetry 1998, 9, 4249-4252) according to the given method:

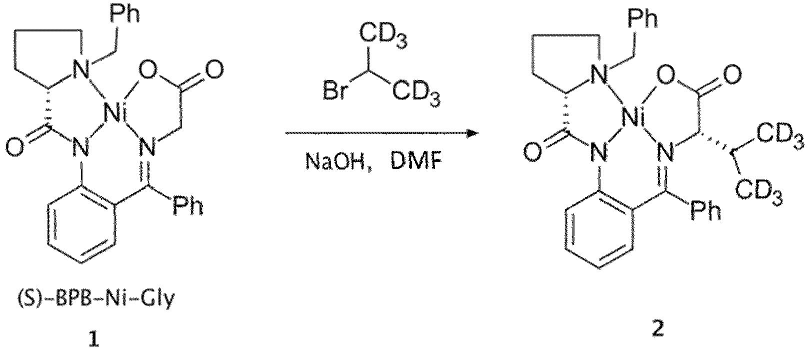

A solution of 10 g (0.02 mol) of complex 1 in 12 ml of dry dimethylformamide (DMF) was cooled under argon to 0 °C. Then, 1 g (0.025 mol) of finely ground NaOH was added to the solution with vigorous stirring and stirred for 5 minutes. Then, 2 ml (2.62 g, 0.021 mol) of isopropyl bromide-d₆ was added dropwise to the solution at 0 ° C and stirred for 30 minutes, after which the temperature was brought to room temperature and stirred for another 1.5 hours. Upon completion of the reaction, the solution was cooled again, then a solution of acetic acid (1.5 ml of ice-cold CH₃COOH in 20 ml of water) was added dropwise to it. During the addition of the acetic acid solution, complex **2** precipitated out. The resulting precipitate was filtered off, washed with water, and dried. Complex **2** was obtained as a red powder (10.5 g, 0.019 mol, 97%).

¹H-NMR (400 MHz, CDCl₃): δ = 8.24 (d, J = 8.6 Hz, 1H), 8.01 (d, J = 7.4 Hz, 2H), 7.56-7.40 (m, 4H), 7.32 (t, J = 7.6 Hz, 2H), 7.20―7.09 (m, 2H), 6.91 (d, J = 7.6 Hz, 1H), 6.68-6.60 (m, 2H), 4.47 (d, J = 12.8 Hz, 1H), 3.82 (d, J = 3.2 Hz, 1H), 3.62 (d, J = 12.6 Hz, 1H), 3.54-3.42 (m, 2H), 3.38-3.28 (m, 1H), 2.88-2.75 (m, 1H), 2.61―2.42 (m, 1H), 2.12-2.01 (m, 2H), 1.79-1.72 (m, 1H).

12 ml of 12M HCl and 18 ml of water was added to a solution of 10.5 g (0.019 mol) of complex **2** in 17 ml of methanol and then the mixture was boiled to boil under reflux for 30 minutes. Then the reaction mixture was evaporated. The formed precipitate of the chiral ligand was filtered off, washed several times with water, and dried. The ligand was obtained as a white powder with 81% yield (6.82 g). The collected aqueous layer was neutralized with 25% ammonia solution, the chiral ligand remaining in the solution was extracted with methylene chloride (3×30 ml), then the aqueous layer was filtered through a paper filter. Valine-d₆ was purified using ion exchange chromatography. For this purpose an aqueous solution was added to the KU-2 ion-exchange resin in H ⁺ - form (13×3 cm). First, the resin column was washed with water (300 ml), then valine-d₆ was washed off the column with 5% ammonia solution (200 ml). The resulting solution was evaporated to dryness, the product was recrystallized from a mixture of water and ethanol. Valine-d₆ was isolated as a white powder (1.37 g, 62%).
¹H-NMR (400 MHz, D₂O): δ = 3.49 (d, J = 4.4 Hz, 1H), 2.16-2.10 (m, 1H).
¹³C-NMR (101 MHz, D₂O) δ = 174.3, 60.3, 28.6, 17.6-15.0 (m).

**Synthesis of valine-2,4,4,4,4 ", 4 ", 4'-D₇ (mixed with valine-4,4,4,4 ", 4", 4'-D₆)** 20 equivalents of methanol-d**1** and 5 mol% of triethylamine were added to a solution of 10 g (0.02 mol) of complex ₁ in 50 ml of methylene chloride and stirred for 18 hours at room temperature. The resulting complex **1-D** was used in the next stage without purification.

¹H-NMR (400 MHz, CDCl₃): δ = 8.30 (d, J = 8.6 Hz, 1H), 8.09 (d, J = 7.3 Hz, 2H), 7.62― 7.49 (m, 3H), 7.44 (t, J = 7.5 Hz, 2H), 7.32 (t, J = 7.4 Hz, 1H), 7.23 (t, J = 7.3 Hz, 1H), 7.12 (d, J = 6.8 Hz, 1H), 7.05―6.95 (m, 1H), 6.82 (d, J = 7.6 Hz, 1H), 6.72 (t, J = 7.4 Hz, 1H), 4.50 (d, J = 12.6 Hz, 1H), 3.73―3.64 (m, 2H), 3.49 (dd, J = 10.7, 5.4 Hz, 1H), 3.44―3.30 (m, 1H), 2.64―2.53 (m, 1H), 2.52―2.36 (m, 1H), 2.22-2.03 (m, 2H).

A solution of 5 g (0.01 mol) of complex **1-D** in 6 ml of dry dimethylformamide (DMF) was cooled to 0° C under argon. Then, 0.5 g (0.0125 mol) of finely ground NaOH was added to the solution with vigorous stirring and stirred for 5 minutes. Then 1 ml (1.31 g, 0.01 mol) of isopropyl bromide-d⁶ was added to the solution dopwise at 0 ∘ C and stirred for 30 minutes, then the temperature was brought to room temperature and stirred for another 1.5 hours. After completion of the reaction (TLC analysis), the solution was cooled again; a solution of acetic acid (1.5 ml of ice-cold CH₃COOH in 20 ml of water) was added dropwise. When adding the acetic acid solution, the resulting **2-D** complex precipitated out.

3 ml of a concentrated HCl solution (12 M solution) and 5 ml of water were added to the solution of 2 g (0.004 mol) of the **2-D** complex in 5 ml of methanol, and then the mixture was boiled to boil under reflux for 30 min. Then the reaction mixture was evaporated. The formed precipitate was washed with water, the chiral ligand was filtered off and washed several times with water. The collected aqueous layer was neutralized with a 25% ammonia solution, the ligand **3** remaining in the solution was extracted with methylene chloride (3×10 ml), then the aqueous layer was filtered through a paper filter and added to the KU-2 ion-exchange resin in the H ⁺ form (13×3 cm). First, the resin column was washed with water (300 ml), and then valine-d₆ / d₇ was washed from the column with 5% ammonia solution (200 ml). Valine-d₆ / d₇ was purified from the glycine impurity by liquid chromatography on silica gel (eluent: dichloromethane-methanol-water). Yield: 172 mg (35%).

¹H-NMR (400 MHz, D₂O): δ = 3.49 (d, J = 4.4 Hz, 1H), 2.16-2.10 (m, 1H).

The ratio of the peaks corresponding to isotopic forms of d₅, d₆ and d₇ is 6.1: 49.5: 44.4 based on mass spectrometric analysis. Form d₅ is the product of incomplete deuteration of methyl groups. Taking into account the contribution of ^{13C} isotope and the equal degree of deuteration of the α position, regardless of the degree of methyl groups deuteration, the total molar fraction of α-deuterated forms of valine in the final product is 49%.

### Synthesis of leucine-5,5,5,5 ', 5', 5'-D₆

### 1) Isobutanol-d₆

4.19 g of magnesium is poured into a two-necked flask, a reflux condenser is connected and fitted to a water-jet pump to evacuate the flask. The flask is heated with hot air for several minutes. After the flask is cooled to room temperature, 20 ml of THF and an iodine crystal are added, purged with argon, and stirred at room temperature for ~ 10 min. A calcium chloride tube is connected to the refrigerator, and a solution of isopropyl bromide-d₆ (22.5 g, 0.17 mol) in 68 ml of THF is added slowly (~ 2.5 h). The mixture heats up and turns gray. After adding the whole isopropyl bromide, the reaction mixture is heated at 50―60 ° C for 2 h. Then the reaction mixture is cooled to room temperature and in portions, without access of air, dry and finely ground paraform is added (5.22 g, 0.17 mol; preliminary dried in vacuum over phosphoric anhydride for two days). The reaction mixture is stirred at 50 ° C for 3 h and then is left overnight at room temperature. The solvent is evaporated to dryness on a rotary evaporator. The dry residue is dissolved in 75 ml of dichloromethane and slowly acidified with a solution of 20% sulfuric acid to a weakly acidic medium. The organic layer is separated, and the aqueous layer is extracted 3 times with 25 ml of dichloromethane. The combined organic extracts are washed with soda solution and dried over sodium sulfate. After filtration, the organic phase is fractionated with a reflux condenser, collecting separately fractions with a boiling range of 85―100 °C and 100―115 C. The first fraction consists of THF and product in a 2.5: 1 ratio. The second fraction contains a small admixture of THF. Total yield: 70%. ¹H NMR spectrum (600 MHz, CDCl₃, ppm): δ = 3.37 (2H, d, ³*J*=6.5 Hz, CH₂), 1.94 (1H, br s, CH).

### 2) Isobutyl bromide-d₆

Isobutanol-d₆ (4.9 g, 0.06 mol) is cooled to -10 ° C and PBr₃ (10.3 g, 0.038 mol) is slowly added dropwise. The reaction mixture is stirred at room temperature overnight. Isobutyl bromide-d₆ is distilled off from the reaction mixture, collecting the fraction boiling at 65―90 °C. The distillate is washed with water and dried over zeolite. Yield: 6.6 g (73%). ¹H NMR spectrum (600 MHz, CDCl₃, ppm): δ = 3.30 (2H, d, ³*J*=6.1 Hz, CH₂), 1.93―1.95 (1H, m, CH).

### 3) Leucine-5,5,5,5 ', 5', 5'-D₆

Leucine-5,5,5,5 ', 5', 5'-D₆ was prepared similarly to valine-d₆ by replacing isopropyl bromide-d₆ with an equivalent amount of isobutyl bromide-d₆. The yield of the final product after recrystallization is 50%.

¹H-NMR (600 MHz, D₂O, internal standard: methanol): δ = 1.64-1.76 (3H, m), 3.71 (1H, dd, ³*J* = 6.0, 8.4 Hz, CH-α).

¹³C-NMR (101 MHz, D₂O, internal standard: methanol) δ = 20.4 (CD₃), 24.31, 40.33, 54.08, 176.24.

In the examples below, a Bruker BioSpec BC70 / 30 USR tomograph with a constant field of 7.05 T was used, equipped with a birdcage coil tuned to ¹H (transmit / receive) and ²H (transmit), and a surface receive coil with a diameter of 5 cm.

The deuterium tomogram was recorded using FLASH (Fast low angle shot) pulse sequence. The excitation frequency was determined from the ²H NMR spectrum and was on the instrument used: sfo1 ≈ 46.17452 MHz, rectangular excitation pulse 2560 Hz wide and 11.2 dB power, deflection angle FA = 30°, repetition time TR = 11.8 ms, echo time TE = 4.07 ms, scan region10 cm × 10 cm, scan matrix 50 × 50, slice thickness 3 cm, bandwidth 12500 Hz, total scan time 9 minutes 34 seconds (1024 accumulations).

### Example 1. Registration of deuterium tomogram and ²H NMR spectrum of a sample containing a dilute solution of deuterated valine.

To demonstrate the fundamental possibility of registering a deuterium tomogram of a diluted solution of deuterated valine, the following experiment was carried out.

Glass vial containing 5 ml of a solution of valine-4,4,4,4 ', 4', 4'-d₆ or leucine-5,5,5,5 ', 5', 5'-d₆ (5 mg) in distilled water, was placed in the center of the tomograph magnet. A surface coil 5 cm in diameter was positioned horizontally directly above the vial.

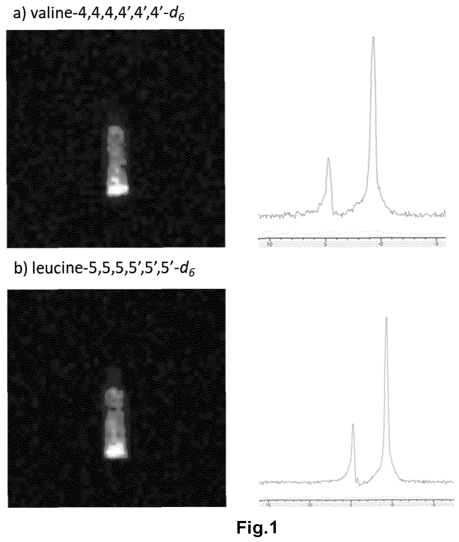

Figure 1a shows the deuterium tomogram (on the left) and a ²H spectrum (on the right) of the sample with valine-4,4,4,4 ', 4', 4'-d₆.
Figure 1b shows the deuterium tomogram (on the left) and a ²H spectrum (on the right) of the sample with leucine-5,5,5,5 ', 5', 5'-d₆.

### Example 2. Use of deuterium tomography to visualize mouse 4T1 breast carcinoma in vivo using the diagnostic drug containing valine-4,4,4,4", 4", 4'-d₆.

In this example, experiments were performed on Balb / c mice grafted with 4T1 breast carcinoma (injection of 5•10⁵ cells / 60 µl under the left forepaw 12 days before the experiment). An animal weighing 20 g was injected intraperitoneally with a solution of 20 mg valine-d₆ in 0.5 ml of water. In 10 minutes after the injection, the animal was immobilized with isoflurane and placed on a heated bed in a tomograph. The surface ²H receiving coil was positioned over the anterior part of the animal's body from the dorsal side, as indicated in figure 2 by the dotted line.

Figure 2 shows that over time, the deuterated valine derivative accumulates in the tumor tissue, with the maximum intensity of the deuterium signal observed about 90 minutes after the drug administration. As can be seen from the data given, the deuterium signal persists for several tens of minutes; thus, valine-d₆ has favorable pharmacokinetics for practical use in ²H MRI and MR spectroscopy.

As follows from the above results, the diagnostic drug based on a deuterated valine derivative can be used for non-invasive diagnosis of diseases, including oncological ones, by ²H MRI and / or MR spectroscopy.

### Example 3. Use of deuterium tomography to visualize mouse 4T1 breast carcinoma in vivo using the diagnostic drug containing leucine-d₆.

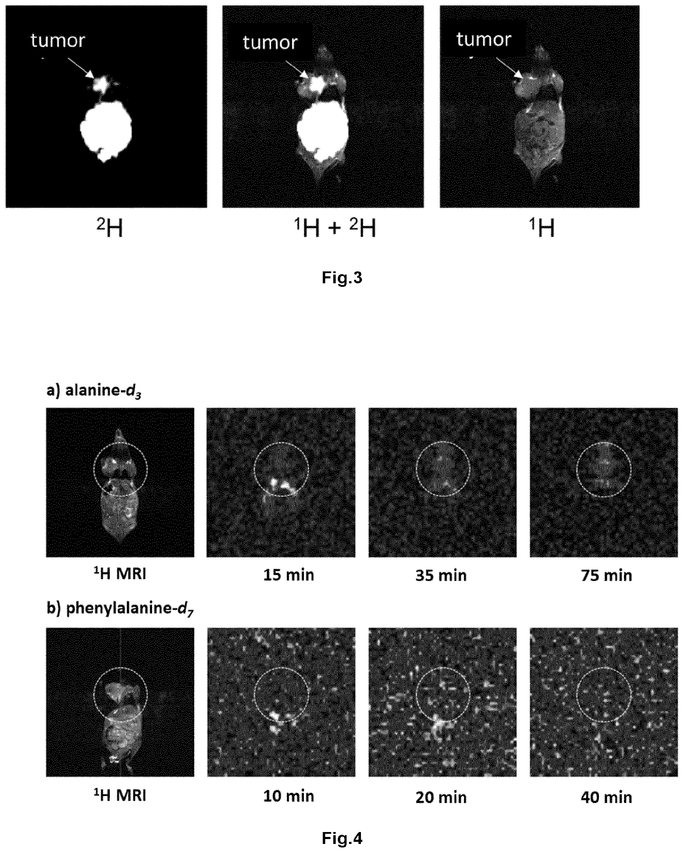

In this example, experiments were performed on Balb / c mice grafted with 4T1 breast carcinoma (injection of 5•10⁵ cells / 60 µl under the left forepaw 12 days before the experiment). An animal weighing 20 g was injected intraperitoneally with a solution of 25 mg of leucine-5,5,5,5 ', 5', 5'-d₆ in 0.8 ml of water. In 10 minutes after the injection, the animal was immobilized with isoflurane and placed on a heated bed in a tomograph. The surface receiving ²H coil was located over the front part of the animal's body from the dorsal side. Figure 3 shows an image obtained 40 minutes after the drug administration.

As follows from the above results, the diagnostic drug based on a deuterated leucine derivative can be used for non-invasive diagnosis of diseases, including oncological ones, by ²H MRI and / or MR spectroscopy.

### Example 4. The use of deuterium tomography for visualization of mouse 4T1 breast carcinoma in vivo using the diagnostic drug containing L-alanine-3,3,3-d₃ and L-phenylalanine -β ,β, 2,3,4,5,6-d₇.

In this example, experiments were performed on Balb / c mice grafted with 4T1 breast carcinoma (injection of 5×10⁵ cells / 60 µl under the left forepaw 12 days before the experiment).

An animal weighing 20 g was injected intraperitoneally with a solution:
a) 30 mg of L-alanine-3,3,3-d₃ in 0.5 ml of water; or
b) 10 mg of L-phenylalanine - β,β,2,3,4,5,6-d₇ in 1.0 ml of water (a lower dose of phenylalanine equivalent to deuterium is due to the low solubility of phenylalanine and, as a consequence, the impossibility of introducing a larger volume of the diagnostic drug without harm for the health of a subject).

In 10 minutes after the injection, the animal was immobilized with isoflurane and placed on a heated bed in a tomograph. The surface ²H receiving coil was positioned over the anterior part of the animal's body from the dorsal side, as indicated in figure 4 by the dotted line.

Figure 4 shows that deuterated derivatives of alanine and phenylalanine do not accumulate in tumor tissue and do not show accumulation selectivity in various organs.

### Example 5. Determination of the content of valine-4,4,4,4',4',4'-d₆ in the tissues of mice with 4T1 breast carcinoma after administration of the diagnostic drug at a dose compatible with deuterium tomography.

In this example, experiments were performed on Balb / c mice grafted with 4T1 breast carcinoma (injection of 5×10⁵ cells / 60 µl under the left forepaw 12 days before the experiment). An animal weighing 20 g was injected intraperitoneally with a solution of 20 mg *L*-valine-d₆ (S-valine-d₆) in 0.5 ml of water. After administration, the animal was kept in a separate cage with access to food and water. After a certain time after administration, the animal was devitalized by dislocation of the cervical vertebrae. Samples of animal organs and tissues were quickly removed and frozen in liquid nitrogen. Frozen samples were ground in a porcelain mortar while cooling with liquid nitrogen. A weighed portion (50-200 mg) of the obtained powder was quickly introduced into 0.4% hydrochloric acid (0.6-1.0 ml) heated to 98 ° C. The resulting suspension was kept at 98 ° C for 15 min with occasional shaking. After adding an internal standard (3-O- (CD₃) -glucose solution), the mixture was shaken and centrifuged until the solid fractions were completely separated. The solution was analyzed by ²H NMR.

**Table 1.**

| | [valine-d₆], µmol / g, 30 min. | Tumor: tissue ratio, 30 min. | [valine-d₆], µmol / g, 1 hour | Tumor: tissue ratio, 1 hour | % dose / g, 1 hour* |
|---|---|---|---|---|---|
| Tumor | 9.2 | - | 9.8 | - | no data |
| Kidneys | 9.3 | 0.99 | 7.5 | 1.3 | 0.55 |
| Liver | 17.9 | 0.51 | 11.5 | 0.85 | 0.55 |
| Heart | 7.5 | 1.2 | 8.0 | 1.2 | no data |
| Brain | 1.6 | 5.8 | 2.6 | 3.8 | no data |
| Spleen | 14.7 | 0.63 | 14.1 | 0.70 | 0.58 |
| Sceletal muscles | 7.4 | 1.2 | 7.7 | 1.3 | 0.16 |
| Lungs | 10.6 | 0.87 | 8.8 | 1.1 | 0.35 |
| Fat tissue | 4.0 | 2.3 | 1.7 | 5.8 | no data |

| | | | | | |
|---|---|---|---|---|---|
| * According to Washburn et al. (1978): Percentage of the administered dose of *DL*-1-¹⁴C-valine (0.021 mg / kg) per 1 gram of corresponding tissue in a healthy rat. | | | | | |

The data presented in Table 1 indicate the selective accumulation of *L*-valine-d₆ in various tissues of the animal in a concentration range suitable for use in ²H MRI or MR spectroscopy. In particular, there is an increased accumulation of *L*-valine-d₆ in tumor tissue compared to kidneys, heart, brain, skeletal muscles and adipose tissue 1 hour after the injection of the diagnostic drug.

Also from Table 1 it can be concluded that there is a significant difference in the relative concentrations of the isotopic label achieved in different organs: ²H in the case of *L*-valine-d₆ (1.25 g / kg) and ¹⁴C in the case of *DL*-1-¹⁴C -valine (0.021 mg / kg). The absolute concentrations of valine in rat liver and kidneys according to Washburn et al. (1978), shown in Table 1, as a result of the introduction of *DL*-1-¹⁴C-valine (0.021 mg / kg) were increased by no more than 0.3 nmol / g compared to the physiological concentration of free valine of about 0.5 µmol / g [Rivera , S. et al. Biochem. J. 1988, 249, 443-449]. In our experiments with valine-d₆, as follows from Table 1, a more than 10-fold excess of the physiological concentration of free valine in animal tissues is achieved.

### Example 6. Study of the distribution and metabolism of the deuterated valine derivatives mixture in the tissues of mice with 4T1 breast carcinoma after administration of the diagnostic drug at a dose compatible with deuterium tomography.

In this example, *L*-valine-d₆ / d₇, partially deuterated at the α position and positions 4, 4', was used. (S-valine-d₆ / d₇). The total molar fraction of α-deuterated forms of valine in the initial diagnostic drug is 49%.

The experiments were carried out on Balb / c mice grafted with 4T1 breast carcinoma (injection of 5×10⁵ cells / 60 µl under the left forepaw 12 days before the experiment). An animal weighing 20 g was injected intraperitoneally with a solution of 20 mg partially deuterated in the α-position of valine in 0.5 ml of water. After administration, the animal was kept in a separate cage with access to food and water. After 60 minutes after administration, the animal was devitalized by dislocation of the cervical vertebrae. Samples of animal organs and tissues were quickly removed and frozen in liquid nitrogen. Frozen samples were ground in a porcelain mortar while cooling with liquid nitrogen. A weighed portion (about 50 mg) of the obtained powder was quickly introduced into 0.4% hydrochloric acid (1.0 ml) heated to 98 °C. The resulting suspension was kept at 98 °C for 15 min with occasional shaking, then centrifuged. The solution was diluted with 3 volumes of acetonitrile, centrifuged after 15 minutes, diluted with 9 volumes of water, and centrifuged again. The ratio of isotopomers d₅, d₆, and d₇ of valine was determined by LC-MS / MS.

The total mole fraction α deuterated valine is: in the tumor - 12%, in the brain - 5%, in the blood - 12%, in the kidneys - 17%, in the spleen - 13%, in the liver, skeletal muscles and adipose tissue - 11%. Thus, by the time a concentration of deuterium in a tumor reaches acceptable one for ²H MRI, there is a significant loss of deuterium from the α-position of valine, presumably as a result of the action of tissue transaminases. Differences in the loss of deuterium from the α-position in different tissues indicate a difference in the ratio of the rates of membrane transport and transamination, which can serve as additional information used in assessing the metabolic status of various tissue sections in a subject and in making a conclusion on the presence or absence of pathology. This result indicates the possibility of using the diagnostic drug containing a mixture of various deuterated derivatives of valine or a derivative of valine with deuterium in several nonequivalent positions.

Although the invention has been described with reference to the disclosed embodiments, it should be apparent to the subject matter experts that the specific experiments described in details are provided for the purpose of illustrating the present invention only and should not be construed as in any way limiting the scope of the invention. It should be understood that various modifications are possible without departing from the spirit of the present invention.

## Claims

1. The diagnostic drug comprising at least one compound selected from a deuterated natural branched-chain amino acid derivative and / or a pharmaceutically acceptable salt of a deuterated natural branched-chain amino acid derivative for the diagnosis of diseases by deuterium magnetic resonance imaging and / or deuterium magnetic resonance spectroscopy.

2. The diagnostic drug according to the claim 1, which additionally comprises at least one pharmaceutically acceptable auxiliary substance.

3. The diagnostic drug according to the claim 2, **characterized by** the fact that the pharmaceutically acceptable excipient is a carrier, filler and / or solvent.

4. The diagnostic drug according to the claim 1, in which the deuterated natural branched-chain amino acid derivative is deuterated valine or deuterated leucine or deuterated isoleucine.

5. The diagnostic drug according to the claim 1 additionally comprising an inhibitor of natural branched-chain amino acid metabolism.

6. The diagnostic drug according to the claim 4, in which the deuterated valine is valine-4,4,4-d₃, valine-4,4,4,4',4',4'-d₆, valine-3,4,4,4,4',4',4'-d₇, valine-2,4,4,4,4',4',4'-d₇, valine-2,3,4,4,4,4',4',4'-d₈.

7. The diagnostic drug according to the claim 4, in which the deuterated leucine is leucine-3,3-d₂, leucine-5,5,5-d₃, leucine-5,5,5,5',5',5'-d₆, leucine-4,5,5,5,5',5',5'-d₇, leucine-3,3,5,5,5,5',5',5'-d₈, leucine-3,3,4,5,5,5,5',5',5'-d₉, leucine-2,5,5,5,5',5',5'-d₇, leucine-2,3,3,5, 5,5,5',5',5'-d₉, leucine-2,4,5,5,5,5',5',5'-d₈, leucine-2,3,3,4,5,5,5,5',5',5'-d₁₀.

8. The diagnostic drug according to the claim 4, when the deuterated isoleucine is 2-amino-3- (CD₃) pentanoic acid, 2-amino-3- (CD₃) pentanoic-5,5,5-d₃ acid, 2-amino-3 - (CD₃) pentanoic-4,4,5,5,5-d₅ acid, 2-amino-3- (CD ₃) pentanoic-2,5,5,5-d₄ acid, 2- amino-3- (CD₃) pentanoic-2,3,5,5,5-d₅ acid, 2-amino-3- (CD₃) pentanoic-3,4,4,5,5,5-d₆ acid, 2- amino-3-(CD₃) pentanoic-2,3,4,4,5,5,5-d₇ acid, 2-amino-3-methylpentanoic-5,5,5-d₃ acid.

9. The diagnostic drug according to the claim 1, when the deuterated derivative is wholly or predominantly represented by one enantiomer.

10. The diagnostic drug according to the claim 9, in which the deuterated derivative is wholly or predominantly represented by the enantiomer with 2S configuration.

11. The diagnostic drug according to the claim 1, in which the deuterated natural branched-chain amino acid derivative is
- a mixture of at least two different deuterated derivatives of valine or its pharmaceutically acceptable salts; or
- a mixture of at least two different deuterated derivatives of leucine or its pharmaceutically acceptable salts; or
- a mixture of at least two different deuterated derivatives of isoleucine or its pharmaceutically acceptable salts; or
- a mixture of at least two different deuterated derivatives of valine, leucine and / or isoleucine and / or their pharmaceutically acceptable salts.

12. The diagnostic drug according to the claim 1, in which the deuterated derivative of a natural branched-chain amino acid and / or its pharmaceutically acceptable salt, along with deuterium atoms bonded to carbon atoms, contain deuterium atoms partially or completely replacing mobile hydrogen atoms bonded to oxygen atoms and / or nitrogen.

13. The diagnostic drug according to the claim 1, which includes
- a deuterated derivative of a natural branched-chain amino acid containing deuterium atoms bonded to carbon atoms in more than one position, or a pharmaceutically acceptable salt thereof; or
- a mixture of deuterated derivatives of natural branched-chain amino acids and / or their pharmaceutically acceptable salts, with deuterated derivatives containing deuterium atoms in structurally nonequivalent positions.

14. The method for diagnosing a disease in a subject, comprising the following steps:
a) the diagnostic drug according to any of the claims 1-13 is administered to a subject;
b) magnetic resonance imaging and / or deuterium magnetic resonance spectroscopy is carried out after the administration of the diagnostic drug in a time sufficient for its accumulation in a target tissue, to obtain, respectively, a tomogram and / or NMR spectrum (spectra);
c) the presence or absence of a disease is diagnosed based on the observed signal intensity of deuterium nuclei, reflecting the level of accumulation of the diagnostic drug.

15. The method according to the claim 14, in which a disease is an inflammatory process, an infectious process, a process accompanied by active regeneration, a disease associated with ischemia of organs and tissues, a graft rejection reaction, an autoimmune disease.

16. The method according to the claim 14, in which a disease is an oncological disease.

17. The method according to the claim 14, in which at least one additional medical examination is performed, selected from magnetic resonance imaging on nuclei other than deuterium nuclei, ultrasound examination, computed tomography, radiography, palpation, biopsy, analysis of biological material of the subject for tumor markers, radionuclide diagnostics and / or visual observation.

18. The method according to the claim 14, when the presence or absence of a disease is diagnosed based on comparing the signal intensity of deuterium nuclei in a subject under examination with the typical signal intensity observed in healthy subjects in a corresponding tissue or corresponding organ.

19. The method according to the claim 14, when the presence or absence of a disease is diagnosed based on a comparison of the deuterium signal intensity in regions corresponding to normal and abnormal tissue according to additional medical examination data.

20. The method according to the claim 14, in which the diagnostic drug is used comprising a deuterated derivative of a natural branched- chain amino acid containing deuterium atoms in more than one position or a mixture of deuterated derivatives of a natural branched- chain amino acids containing deuterium atoms in structurally nonequivalent positions, and in which the level of metabolic activity and / or proliferation of cells of a target tissue is additionally assessed based on a comparison of the intensity of signals of deuterium nuclei in structurally nonequivalent positions.

21. The method according to the claim 17, when the presence or absence of a disease is diagnosed based on the comparison of the deuterium tomogram of a subject under examination with the image obtained as a result of protium magnetic resonance imaging of a subject.

22. The method according to the claim 16, when the spatial distribution of the region with increased accumulation of the diagnostic drug is used to make a conclusion about the structure of a tumor.

23. The method according to the claim 16, in which the intensity of the deuterium signal in the region of increased accumulation of the diagnostic drug is used to make a conclusion about the malignancy or benignity of a tumor.

24. The method according to the claim 14, when the rate of deuterium signal intensity change after injection is used to make a conclusion about the level of perfusion in different parts of the scanned region.

25. The method according to the claim 14, when the diagnostic drug is administered to a subject per os.

26. The method according to the claim 14, when the diagnostic drug is administered to a subject parenterally.
